# EUROPÄISCHE PATENTSCHRIFT

(11) **EP 1 870 033 B1**
(45) Veröffentlichungstag und Bekanntmachung des Hinweises auf die Patenterteilung: **28.09.2011**
(21) Anmeldenummer: 07110811.2
(22) Anmeldetag: 21.06.2007
(51) Int. Cl.: A61B 5/00, G01N 33/49

(54) **Vorrichtungen und Verfahren zum Nachweisen eines Analyten**
Devices and method for detecting an analyte
Dispositifs et procédés destinés à la détermination d'un analyte

(30) Priorität: 21.06.2006 EP 06115843
(43) Veröffentlichungstag der Anmeldung: 26.12.2007
(73) Patentinhaber: Trace Analytics GmbH, 38106 Braunschweig (DE)
(72) Erfinder: Künnecke, Wolfgang, 38102, Braunschweig (DE); Hartlep, Michael, 38102, Braunschweig (DE); Giesenberg, Jens, 38304, Wolfenbüttel (DE)
(74) Vertreter: Eisenführ, Speiser & Partner

(56) Entgegenhaltungen:
- EP-A- 0 940 151
- EP-A1- 0 367 752
- EP-A2- 0 098 550
- EP-A2- 0 251 027
- EP-A2- 0 475 534
- WO-A2-2005/042059
- US-A- 3 661 010
- US-A- 3 983 864
- US-A- 4 221 567
- US-A1- 2006 009 727

## Beschreibung

Die Erfindung betrifft Vorrichtungen und Verfahren zum Nachweisen eines Analyten.

In der Verfahrenstechnik und Medizin ist es häufig notwendig, das Vorhandensein und gegebenenfalls die Konzentration eines vorgewählten Analyten schnell und möglichst kontinuierlich zu bestimmen. Hierzu sind eine Reihe von Sonden und Sensoren im Gebrauch. Beispielsweise ist aus der WO 00/25107 eine Tauchsonde bekannt, die in ein analytenhaltiges Medium - beispielsweise das Innere eines Bioreaktors - eingetaucht wird und darin einen Analyten nachweist. Die Tauchsonde ist vom zu analysierenden Medium durch eine Membran getrennt, die für den Analyten durchlässig ist. Es hat sich jedoch als nachteilig insbesondere bei der Überwachung eines Bioreaktors herausgestellt, dass bei dieser Ausgestaltung der Analysenvorrichtung eine Verdünnung des zu analysierenden Analyten nicht mehr möglich ist. Dementsprechend können nur solche Sensoren sinnvoll eingesetzt werden, deren maximaler Messbereich größer ist als die höchst zu erwartende Analytkonzentration. Ansonsten würde der Sensor unabhängig von der tatsächlichen Analytkonzentration stets den maximalen Messwert anzeigen. Bei dieser Analysevorrichtung ist daher der Messbereich, innerhalb dessen die Konzentration des Analyten bestimmt werden kann, begrenzt.

Dies ist besonders nachteilig bei der Überwachung biotechnologischer und medizinischer Prozesse, beispielsweise bei einer kontinuierlichen Überwachung des Blutzuckergehaltes eines Diabetes-Patienten. Insbesondere bei Diabetes-Patienten kann die Blutzuckerkonzentration binnen kurzer Zeit erheblichen Schwankungen unterworfen sein, die ein rasches, jedoch der jeweiligen Blutzuckerkonzentration genau angepasstes Verabreichen von Insulin erforderlich macht. Dementsprechend besteht gerade in der Medizin und Biotechnologie ein steter Bedarf nach schnellen, empfindlichen und genauen Analyseverfahren für vorgewählte Analyten und entsprechenden Vorrichtungen.

In diesem Zusammenhang offenbart die EP 0 441 179 A1 eine tragbare Analysevorrichtung zum kontinuierlichen Bestimmen der Glukosekonzentration im Blut eines Diabetes-Patienten über einen Zeitraum von 24 bis 36 Stunden. Die Analysevorrichtung umfasst eine Mikrodialysenadel, die über einen Venenkatheter in eine Vene des zu überwachenden Patienten eingeführt wird. Die Mikrodialysenadel umfasst eine glukosedurchlässige Dialysemembran, so dass aus dem Blut des Patienten Glukose in einen hinter der Dialysemembran liegenden Raum diffundieren kann. Dieser Raum wird kontinuierlich durch eine Dialyseflüssigkeit durchspült, wobei die glukosehaltige Dialyseflüssigkeit einem Sensor zugeführt wird. Der Sensor ist in einer tragbaren Vorrichtung untergebracht, die auch einen Vorrat an Dialyseflüssigkeit und eine Aufnahme verbrauchter Dialyseflüssigkeit enthält. Der Sensor ist mit der Mikrodialysenadel über einen sehr dünnen Schlauch mit einem Innendurchmesser von 0,1 mm verbunden, um einen raschen Transport von Glukose aus der Mikrodialysenadel zum Sensor zu ermöglichen. Nachteilig an dieser Vorrichtung ist jedoch, dass der dünne Schlauch einen sehr hohen Transportwiderstand und damit Druck erzeugt, so dass die Gefahr besteht, dass die Dialysemembran oder der Schlauch reißen. Nachteilig ist ferner, dass keine Möglichkeit vorgesehen ist, während des Betriebs der Analysevorrichtung einen Nulllinienabgleich des Sensors vorzunehmen. Es besteht deshalb die Gefahr, dass die Messgenauigkeit des Sensors bei langen Einsatzzeiten beeinträchtigt wird.

Aus der US 6,852,500 B1 ist ein Analyseverfahren zum Bestimmen der Glukosekonzentration in einer Körperflüssigkeit bekannt. Dabei wird durch eine Dialysezelle, die auf einer Seite mit der glukosehaltigen Körperflüssigkeit in Kontakt steht, pulsweise eine Dialyseflüssigkeit zu einem Sensor gepumpt, die in einem Puls glukosefrei ist und dementsprechend über die Dialysemembran Glukose aus der Körperflüssigkeit aufnehmen kann, und in einem weiteren Puls eine bekannte Glukosekonzentration enthält. Auf diese Weise sollen Messungen der Glukosekonzentration in der zu analysierenden Körperflüssigkeit im Abstand von jeweils 9 Minuten möglich werden. Nachteilig an diesem Verfahren ist zum einen die mit 9 Minuten relativ langsame Taktzeit, zum anderen jedoch auch die aufwendige Steuerung des Verfahrens und der durch wiederholte Verabreichung einer Dialyselösung mit bekannter Glukosekonzentration hohe Verbrauch an Dialyseflüssigkeit.

Die DE 27 37 922 A1 wiederum offenbart eine Analysevorrichtung zum Überwachen einer Blutglukosekonzentration. Die Analysevorrichtung besitzt wiederum eine Dialysemembran zum Abtrennen von Glukose von weiteren Blutbestandteilen. Die Dialyseflüssigkeit soll dabei im Kreislauf über einen enzymatischen Glukosesensor (Glukoseoxedase mit Sauerstoffelektrode) durch die Dialysemembran geführt werden. Gegebenenfalls kann pulsweise eine Kalibierlösung zudosiert werden. Nachteilig ist jedoch, dass durch den geschlossenen Kreislauf der Dialyseflüssigkeit während des üblichen Betriebes einer solchen Vorrichtung ein vollständiger Ausgleich der Konzentration von Glukose in der Dialyseflüssigkeit und dem Blut eines überwachten Patienten stattfinden, so dass der Sensor wiederum mit zum Teil sehr hohen Glukosekonzentrationen beaufschlagt wird. Dementsprechend muss der Sensor einen sehr breiten Messbereich besitzen, oder entsprechend unempfindlich sein. Durch die hohe Beanspruchung des Sensors besteht ferner die Gefahr, dass dieser rasch inaktiviert wird.

EP0251027 beschreibt eine Vorrichtung zur Bestimmung des Partialdrucks von einem in Fluid gelösten Gasen und Gasgemischen. Die Vorrichtung enthält einen Katheter, in dem ein Trägerfluid mit einem Analyten angereichert wird, und eine Messkammer. Sie enthält außerdem einen Bypass, mittels dessen es möglich ist einen Flüssigkeitsstrom abzuzweigen und ohne Passieren des Katheters direkt in die Messeinrichtung zu leiten.

Die US 4,245,634 offenbart eine Analysevorrichtung in Form einer künstlichen Bauchspeicheldrüse, bei der über einen doppellumigen Katheter durch Infusion mit Heparinlösung verdünntes Blut einem Patienten entnommen und die Blutglukosekonzentration photometrisch bestimmt wird. Nachteilig daran ist, dass die Messgenauigkeit abhängt von der Pumpgeschwindigkeit der infundierten Heparinlösung. Zudem sind keine Kalibriermöglichkeiten vorgesehen.

Die US 2006/0009727 A1 offenbart eine Analysevorrichtung zum extrakorporalen Bestimmen einer Blutglukosekonzentration. Dabei wird in einem Kreislauf Blut durch einen doppellumigen Katheter einem Patienten entnommen und über eine Ultrafiltratitonseinheit ein glukosehaltiges Ultrafiltrat abgetrennt, das einem Glukosesensor zugeführt wird. Das Ultrafiltrat wird gesammelt und dem Patienten von Zeit zu Zeit reinfundiert, um den Volumenverlust von Blutflüssigkeit gering zu halten. Zum Kalibrieren wird dabei eine Kalibrierflüssigkeit über einen Kalibriersensor geleitet. Während der Kalibriersensor im Regelbetrieb vom Ultrafiltrat bespült wird, wird zum Kalibrieren die Fließrichtung umgekehrt, das Ultrafiltrat zurück in die Ultrafiltrationseinheit gepresst und der Kalibriersensor mit Kalibrierflüssigkeit beaufschlagt. Nachteilig hieran ist der komplizierte Aufbau der Vorrichtung und die Notwendigkeit, zum Kalibrieren das Ultrafiltrat zurück in die Ultrafiltrationseinheit zu pressen.

Es war deshalb eine Aufgabe der vorliegenden Erfindung, eine Vorrichtung und ein Verfahren anzugeben zum Detektieren eines Analyten. Die Vorrichtung sollte möglichst einfach aufgebaut sein, sie sollte leicht zu warten sein, sie sollte eine rasche Messung einer Analytkonzentration ermöglichen, möglichst für den Einsatz bei der Behandlung eines Menschen oder Tieres geeignet sein und/oder eine einstellbare Empfindlichkeit besitzen.

Erfindungsgemäß wird deshalb eine Analysevorrichtung angegeben wie in Anspruch 1 beschrieben.

Die erfindungsgemäße Vorrichtung ermöglicht es auf einfache Weise, das Flüssigkeitsvolumen zwischen Analytaufgabekammer und Sensorkammer gering zu halten und dementsprechend eine rasche Analytkonzentrationsbestimmung durchzuführen. Zudem kann die Sensorkammer und die Analytaufgabekammer mit einem sehr geringen Volumen einer Spülflüssigkeit vollständig durchspült werden, was ebenfalls zu einer raschen Messung einer Analytkonzentration beiträgt. Darüber hinaus ermöglicht es die Vorrichtung, beim Spülen der Sensorkammer durch Verwendung einer entsprechend kalibrierten Lösung auch den Sensor neu zu kalibrieren, und so die Messgenauigkeit der erfindungsgemäßen Vorrichtung dauerhaft hoch zu halten. Die erfindungsgemäße Vorrichtung ist zudem sehr einfach aufgebaut und dementsprechend leicht zu bedienen und zu warten.

Durch die Verwendung schaltbarer Absperrungen ist es möglich, die Fließrichtung des Transportmediums in der Sensorkammer umzukehren, ohne die Pumprichtung einer zum Bewegen des Transportmediums allfällig vorhandenen Pumpe umkehren zu müssen. Dadurch gelingt es, das Transportmedium stets nur durch Sog oder nur durch Überdruck im Verteiler und entsprechend auch in der Sensorkammer zu bewegen. Dies vermeidet die bei einer Umkehrung der Pumprichtung auftretenden starken Druckschwankungen des Transportmediums und eine damit einhergehende starke Belastung des oder der Sensoren der Sensorkammer und/oder einer Membran der Analytaufgabekammer. Vorrichtungen, in denen notwendigerweise eine Umkehr der Pumprichtung oder andere starke Druckschwankungen des Transportmediums auftreten, sind beispielsweise offenbart in den Schriften US 4,221,567, EP 0 940 151 A1, EP 0 367 752 A1 und US 3,983,864. Vorteilhaft ist zudem, dass mit einem Verteiler wie erfindungsgemäß vorgesehen auf ein wartungsanfälliges Drehventil verzichtet werden kann, wie es beispielsweise in der EP 0 098 550 A2, WO 2005/042059 A2 und EP 0 251 027 A verwendet wird.

Insbesondere kann der Verteiler und optional auch die Analytaufgabekammer und/oder die Sensorkammer als Einwegartikel ausgeführt sein. Dazu ist der Verteiler, die Analytaufgabekammer bzw. die Sensorkammer vorzugsweise als eigenständiges Bauteil ausgebildet, das mit den übrigen Elementen der erfindungsgemäßen Vorrichtung verbindbar ist. Wenn der Verteiler, die Analytaufgabekammer bzw. die Sensorkammer verschlissen ist, kann das jeweilige Bauteil einfach ausgewechselt und ein rascher Weiterbetrieb der erfindungsgemäßen Vorrichtung erreicht werden.

In bevorzugten Ausführungsformen der erfindungsgemäßen Analysevorrichtung ist oder umfasst jede der schaltbaren Absperrungen zwischen zwei benachbarten Anschlüssen ein durch Druck eines Steuerfluids oder Magnetismus schaltbares Ventil.

Ein solchermaßen ausgerüsteter Verteiler kann aus vorteilhaften wenigen Einzelbauteilen zusammengesetzt werden. Er benötigt nur geringe Abmessungen und erfordert nur wenig Zeit zum Schalten. Er ist leicht zu reinigen und zu warten. Es ist zudem leicht zu sterilisieren und besonders geeignet, das Transportmedium steril zu halten.

Besonders bevorzugt ist ein Verteiler mit einer Absperrung, die eine Membran zum Verschließen des ringförmigen Kanals an der jeweiligen Absperrung umfasst. Vorzugsweise besitzt jede der Absperrungen eine entsprechende Membran. Besonders bevorzugt ist es, wenn die Membran allen Absperrungen gemeinsam und einstückig ist. Eine Membran kann besonders einfach über einen Steuerkanal mit einem Druck eines Steuerfluids - vorzugsweise Druckluftbeaufschlagt und so sehr leicht, schnell und wartungsarm geschaltet werden. Die Absperrungen des erfindungsgemäßen Verteilers kommen in besonders bevorzugten Ausführungsformen bis auf die bewegliche Membran ohne bewegliche Teile aus und sind dann besonders wartungsarm.

Der Verteiler besitzt dementsprechend vier Schaltpunkte zum Ermöglichen bzw. Verbinden des Durchflusses des Transportmediums durch den jeweiligen Schaltpunkt des ringförmigen Kanals. Zweckmäßigerweise ist der Verteiler in zumindest zwei Verteilerzustände schaltbar. In einem ersten, Spül-Schaltzustand sind ein Paar Schaltpunkte geschlossen, wobei jeweils zwischen zwei geschlossenen Schaltpunkten ein geöffneter Schaltpunkt angeordnet ist. Der Verteiler ist so, zusammen mit der Sensorkammer und der Analytaufgabekammer, in einer Spülrichtung in einem ersten Abschnitt des ringförmigen Kanals durchströmbar von einem Zulauf durch einen geöffneten Schaltpunkt in die Sensorkammer und die Analytaufgabekammer, und von dort in einem zweiten Abschnitt des ringförmigen Kanals durch einen weiteren geöffneten Schaltpunkt in einen Ablauf. In einem zweiten, Analyse-Schaltzustand sind jeweils die Schaltpunkte geschlossen, die im Spül-Schaltzustand geöffnet waren, und die Schaltpunkte geöffnet, die im Spül-Schaltzustand geschlossen waren. Im Analyse-Schaltzustand sind Verteiler, Analytaufgabekammer und Sensorkammer entsprechend durchströmbar vom Zulauf durch einen geöffneten Schaltpunkt in die Analytaufgabekammer, von dort in die Sensorkammer und von dort durch einen weiteren Abschnitt des ringförmigen Kanals und durch den weiteren geöffneten Schaltpunkt in den Ablauf.

In einer besonders bevorzugten Ausführungsform umfasst der Verteiler einen Kanal-Grundkörper mit dem darauf angeordneten ringförmigen Kanal. Der ringförmige Kanal ist zu der vom Kanal-Grundkörper abgewandten Seite zumindest an den Stellen, an denen eine Schaltung erfolgen soll, geöffnet, und ist vorzugsweise über seine gesamte Länge auf der vom Kanal-Grundkörper abgewandten Seite geöffnet. Der ringförmige Kanal ist mit einer Membran bedeckt (wiederum zumindest an den zu schaltenden Stellen oder über die gesamte Länge des ringförmigen Kanals). Auf der vom Kanal-Grundkörper abgewandten Seite ist die Membran druckschlüssig verbunden mit einem Schalt-Grundkörper. Der Schalt-Grundkörper besitzt zwei Kanäle mit jeweils zwei Schaltöffnungen, die den jeweiligen Kanal an einem Schaltpunkt des Kanal-Grundkörpers mit der Membran verbinden. Durch Beaufschlagen eines Schaltkanals mit einem unter Druck stehenden Medium, beispielsweise Druckluft, kann die Membran an den Schaltpunkten in den Kanal des Kanal-Grundkörpers hineingepresst und der Kanal an den Schaltpunkten verschlossen werden. Auf diese Weise kann erreicht werden, dass Flüssigkeit den Kanal des Kanal-Grundkörpers auf einfache, vorwählbare Weise in einer von zwei Richtungen durchströmt.

In einer weiteren bevorzugten Ausführungsform umfasst der Verteiler einen prismatischen und vorzugsweise quaderförmigen Kanal-Grundkörper, auf dem zwei Hälften des ringförmigen Kanals auf benachbarten oder vorzugsweise gegenüberliegenden Seiten des Kanal-Grundkörpers angeordnet sind. Die beiden Hälften des ringförmigen Kanals sind dann durch Bohrungen oder Kanalabschnitte miteinander verbunden. Beide Seiten des Kanal-Grundkörpers, die jeweils eine Hälfte des ringförmigen Kanals aufweisen, sind jeweils mit einem Schalt-Grundkörper versehen, der wie oben beschrieben über eine Membran an den Schaltpunkten mit dem Kanal-Grundkörper zum Verschließen der Schaltpunkte wechselwirken kann.

In einer weiteren bevorzugten Ausführungsform umfasst der Verteiler einen Kanal-Grundkörper, auf oder in dem der ringförmige Kanal angeordnet ist. Die Absperrungen werden gebildet durch schaltbare Magnetventile. Ferner umfasst der Verteiler einen Schalt-Grundkörper mit Steuerverbindungen der Magnetventile zur Steuerung.

Ungeachtet der jeweiligen Ausführungsform des Verteilers besitzt der ringförmige Kanal eines Kanal-Grundkörpers und vorzugsweise ferner alle Kanäle, die den ringförmigen Kanal mit der Analytaufgabekammer und der Sensorkammer und die Sensorkammer mit der Analytaufgabekammer verbinden, vorzugsweise einen Innendurchmesser von 0,01 bis 1 mm und besonders bevorzugt von 0,7 bis 0,9 mm, und ermöglicht bzw. ermöglichen so einen Durchfluss eines wässrigen Transportmediums, vorzugsweise wie unten beschrieben, von 1-1,5 ml pro Minute. Bei Verwendung von Kanälen mit rechteckigem oder anderem Querschnitt ist die Querschnittsfläche vorzugsweise bemessen wie bei einem Kanal mit einem Innendurchmesser von 0,01 bis 1 mm und vorzugsweise von 0,7 bis 0,9 mm. Zweckmäßigerweise sind die mit dem Transportmedium in Kontakt stehenden Wände der Kanäle und der Sensor- und Analytaufgabekammer inert gegenüber dem Analyten und verhindern zudem eine nennenswerte Adsorption des Analyten an den Wänden.

Bei Verwendung eines Kanalquerschnitts von 0,01 bis 1 mm und insbesondere von 0,7 bis 0,9 mm können sehr scharf aufgelöste peakförmige Analyt-Konzentrationspulse von der Analytaufgabekammer zur Sensorkammer transportiert und dort vermessen werden. Die Konzentrationspulse bewirken dann die Erzeugung eines peakförmigen Signals durch den Sensor der Sensorkammer. Das Signal des Sensors kann über die Höhe, Fläche oder Steigung des Signals bzw. der Signalflanken ausgewertet werden. Zudem werden bei den genannten Innendurchmessern bzw. den entsprechenden Kanalquerschnittsflächen nur geringe Mengen des Transportmediums für einen Analyttransport von der Analytaufgabekammer zur Sensorkammer benötigt. Werden zudem die Sensorkammer und die Analytaufgabekammer nahe beieinander angeordnet, so dass die Länge des verbindenen Kanals gering ist, so wird ein besonders schnelles Ansprechverhalten des Sensors erreicht, so dass eine hohe Messfrequenz erzielt werden kann. Bei einem Kanal-Innendurchmesser von 100 µm können beispielsweise Fließraten von 10 bis 100 µl/min eingestellt werden, um so ein besonders schnelles Transportieren des Analyten aus der Analytaufgabekammer in die Sensorkammer bei geringem Verbrauch an Transportmedium zu erzielen. Eine bevorzugte implantierbare Analytaufgabekammer wiederum ist mit der Sensorkammer durch einen Kanal mit einem Innendurchmesser von 10 µm verbunden, wobei in diesem Fall Fließraten von wenigen Nanolitern pro Minute für einen Analyttransport zur Sensorkammer ausreichend sind.

Vorzugsweise ist der Verteiler als Einweg-Bauteil ausgeführt und mit den übrigen Elementen der erfindungsgemäßen Vorrichtung durch lösbare Anschlüsse wie oben beschrieben verbindbar. Das entsprechende Einweg-Bauteil umfasst vorzugsweise den gesamten ringförmigen Kanal des erfindungsgemäßen Verteilers, insbesondere bevorzugt in Form eines Kanal-Grundkörpers wie oben beschrieben. In ferner bevorzugten Ausführungsformen umfasst das Einweg-Bauteil ferner die eine oder mehrere Membranen zum Verschließen der Schaltpunkte des Verteilers und einen oder mehrere Schalt-Grundkörper, insbesondere wie oben beschrieben. Die Membran(en) und der oder die Schalt-Grundkörper können dabei fest mit dem Kanal-Grundkörper verbunden oder als mit diesem verbindbare Bauteile ausgeführt sein.

Vorzugsweise umfasst ein als Einweg-Bauteil ausgebildeter Verteiler zusätzlich eine Sensorkammer und/oder eine Analytaufgabekammer.

In bevorzugten Ausführungsformen der Erfindung umfasst die Sensorkammer einen, zwei, drei oder mehr Sensoren.

Jeder Sensor der Sensorkammer einer erfindungsgemäßen Vorrichtung ist vorzugsweise ausgewählt aus der Gruppe bestehend aus elektrochemischen Sensoren und optischen Sensoren, insbesondere amperometrische Sensoren, Leitfähigkeitssensoren, potentiometrische Sensoren, Biosensoren, Sauerstoffsensoren und Enzymsensoren. Die erfindungsgemäße Vorrichtung erhält durch Verwendung eines entsprechenden Sensors einen sehr breiten Anwendungsbereich und ist vielfältig verwendbar. Durch die Auswahl des Sensors ist die Vorrichtung an den jeweiligen Analyten und die zu erwartende Analytkonzentration anpassbar.

Ferner bevorzugt ist es, wenn die Analytaufgabekammer einen oder mehrere Sensoren wie oben beschrieben umfasst. Auf diese Weise lassen sich Analysen unmittelbar in der Analytaufgabekammer durchführen. Der oder die Sensoren können auf der Seite des zu analysierenden Mediums und/oder des Transportmediums angeordnet sein. Soweit ein Sensor auf der Seite des zu analysierenden Mediums angeordnet ist, ist er vorzugsweise sterilisierbar oder durch einen Sterilfilter gegenüber dem zu analysierenden Medium abgegrenzt. Auf diese Weise können Kontaminationen des zu analysierenden Mediums durch den oder die Sensoren verhindert werden.

Die Analytaufgabekammer definiert ein Volumen des Transportmediums und ermöglicht den Übertritt des Analyten aus einem zu analysierenden Medium außerhalb der Analytaufgabekammer in das Transportmedium innerhalb der Analytaufgabekammer. In einer bevorzugten erfindungsgemäßen Vorrichtung ist die Analytaufgabekammer mit einem zu analysierenden Medium über eine Membran verbunden, die den Durchtritt des Analyten aus dem zu analysierenden Medium in das Transportmedium ermöglicht und andere Substanzen von Übertritt das Transportmedium abhält. Die Analytaufgabekammer kann insbesondere die Gestalt einer Dialysekammer haben. Die Membran ist vorzugsweise eine Dialysemembran oder Filtermembran zum Abtrennen des Analyten von dem zu analysierenden Medium. Die Analytaufgabekammer kann beispielsweise und bevorzugt als Tauchsonde mit einer in das zu analysierende Medium eintauchbaren Membran ausgebildet sein. Die erfindungsgemäße Vorrichtung ermöglicht es somit, praktisch ohne Flüssigkeitsverlust aus dem zu analysierenden Medium eine Probe des Analyten zu nehmen. Erfindungsgemäß wird vorzugsweise eine Membran verwendet, die den Durchtritt von Molekülen bis zu einer Größe von 50000 Da und besonders bevorzugt bis zu einer Größe von 12000 Da ermöglicht. Die Analytaufgabekammer kann ebenfalls eine Gasdiffusionsmembran enthalten zum Abtrennen des Analyten von einem zu analysierenden gasförmigen Medium. Die Gasdiffusionsmembran ist vorzugsweise eine poröse Polypropylen- oder Teflon-Membran.

Durch die Verwendung einer entsprechenden Membran in der Analytaufgabekammer besonders bevorzugte Analyten wie Glucose, Fructose, Saccharose, Glycerin, Methanol, Glutamin, Glutamat, Phosphat, Lactat, Lactose, Ammonium, Ethanol, eines Antikörpers, Pestizids, Antibiotikums, Proteingehalts, Fettgehalts, Wassergehalts, pO₂ pCO₂ und/oder pH besonders gut bestimmt werden. Besonders bevorzugte zu analysierende Medien sind flüssige mikrobiologische Medien, insbesondere ein Nährmedium eines Bioreaktors, und physiologische Medien, insbesondere Körperflüssigkeiten wie Blut und Lymphe. Ferner bevorzugte zu analysierende Medien sind gasförmige Medien wie Atemluft oder eine Zu- oder Abluft eines Bioreaktors.

Die Analytaufgabekammer besitzt in bevorzugten Ausführungsformen einen Kanal mit einer Breite von 1,1-1,5 mm, gemessen an der Kontaktstelle zur (Dialyse- oder Gasdiffusions-)Membran, und eine Tiefe von 0,3 bis 1 mm, vorzugsweise beträgt das Kanalvolumen unterhalb der Membran 7 bis 35 µl. Falls der Analyt im Kanal der Analytaufgabekammer oder an sonstigen Orten der erfindungsgemäßen Vorrichtung zur Bildung von Verstopfungen oder Plaques neigt, werden entsprechend größere Kanaldurchmesser und Volumina der Analytaufgabekammer und/oder Sensorkammer gewählt.

Ebenfalls bevorzugt ist eine Analytaufgabekammer in Form einer mit dem Transportmedium durchspülbaren Hohlfaser bzw. ein Bündel durchspülbarer Hohlfasern. Die Hohlfaser(n) wird bzw. werden in ein umgebendes, zu analysierendes vorzugsweise flüssiges oder gasförmiges Medium eingetaucht,
wobei der Analyt durch die Faserwand in das Transportmedium übertreten kann. Zur Ausbildung geeigneter Analytaufgabekammern wird sich der Fachmann vorzugsweise an den in der Hämodialyse oder den in Blut-Oxygenatoren üblichen Hohlfaserbündeln orientieren.

Während der Probenahme aus dem zu analysierenden Medium kann die Analytaufgabekammer mit dem Transportmedium durchspült werden. Dies ist insbesondere dann vorteilhaft, wenn die Analytkonzentration im Transportmedium gering gehalten werden soll. Die Analytaufgabekammer kann jedoch auch mit einem stehenden Transportmedium gefüllt sein, sodass der Analyt in der Analytaufgabekammer angereichert wird. Anhand der Strömungsgeschwindigkeit bzw. der Verweilzeit des Transportmediums in der Analytaufgabekammer kann dann die Konzentration des Analyten in dem zu analysierenden Medium berechnet werden. Die erfindungsgemäße Vorrichtung ermöglicht es dementsprechend auf besonders einfache Weise, den Analyten vor dem Transport in die Sensorkammer auf einen vorgewählten Konzentrationsbereich zu verdünnen oder aufzukonzentrieren, indem die Analytaufgabekammer jeweils mit einer vorgewählten Fließgeschwindigkeit bzw. einer vorgewählten Verweilzeit des Transportmediums beaufschlagt wird. Die erfindungsgemäße Vorrichtung ist dementsprechend geeignet, auch bei rasch stark wechselnden Analytkonzentrationen des zu analysierenden Mediums zuverlässige Analytkonzentrationsbestimmungen vorzunehmen, ohne dass das zu analysierende Medium selbst verdünnt oder ein veränderter Sensor verwendet werden müsste. Die erfindungsgemäße Vorrichtung ermöglicht deshalb besonders präzise Messungen in einem breiten Konzentrationsbereich eines Analyten.

In bevorzugten Ausführungsformen sind die Sensorkammer und der Verteiler in einem einheitlichen Baukörper enthalten. Die erfindungsgemäße Vorrichtung ist besonders einfach miniaturisierbar und dementsprechend für zahlreiche Anwendungszwecke einsetzbar. Die erfindungsgemäße Vorrichtung kann insbesondere verwendet werden zum Beobachten (monitoring) eines Konzentrationsverlaufs beispielsweise in einem Bioreaktor oder an einem Patienten, insbesondere einem Mensch oder Tier, beispielsweise zur Bestimmung der Blutglukose-Konzentration. Die erfindungsgemäße Vorrichtung ist durch die Verwendung einer Analytaufgabekammer mit einer Membran, die den Durchtritt des Analyten aus dem zu analysierenden Medium in das Transportmedium ermöglicht, vorteilhaft einfach sterilisierbar und steril zu halten.

Die erfindungsgemäße Vorrichtung und insbesondere der einheitliche Baukörper ist besonders gut miniaturisierbar. Vorzugsweise besitzt die erfindungsgemäße Vorrichtung deshalb einen einheitlichen Baukörper mit einem Außenmaß des Baukörpers von bis zu 4 x 8 x 3 cm, vorzugsweise 2 x 3 x 0,5 cm wobei als Baukörper dasjenige Bauteil gerechnet wird, das die Sensorkammer und den Verteiler nebst Steuerkanälen für die Schaltpunkte des Verteilers enthält, jedoch ohne Anschlüsse und Zu- oder Ableitungen zu dem Verteiler.

Erfindungsgemäß wird zudem ein Analyseverfahren angegeben zum Betrieb einer erfindungsgemäßen Vorrichtung, umfassend das Pumpen eines Transportmediums in eine Pumprichtung, und mit den Schritten:
- Spülen eines Sensors für die zu analysierende Substanz mit dem Transportmedium in einer ersten Fließrichtung, wobei das Transportmedium nach dem Spülen des Sensors in eine Analytaufgabekammer geleitet wird,
- Beladen des Transportmediums in der Analytaufgabekammer mit einer Menge des nachzuweisenden Analyten und
- ohne Umschalten der Pumprichtung Umkehren der Fließrichtung des Transportmediums, um das gegebenenfalls mit Analyten beladene Transportmedium aus der Analytaufgabekammer zum Sensor zu leiten.

Durch die Verwendung des erfindungsgemäßen Verfahrens können die oben beschriebenen Vorteile der erfindungsgemäßen Vorrichtung erreicht werden. Insbesondere wird nur eine geringe Flüssigkeitsmenge benötigt zum Beaufschlagen des Sensors mit dem analythaltigen Transportmedium, aber auch zum Spülen des Sensors. Das erfindungsgemäße Verfahren ermöglicht so rasche Messungen der Konzentration eines Analyten in einem zu untersuchenden Medium. Durch das Einstellen der Fließgeschwindigkeit oder der Verweilzeit des Transportmediums in der Analytaufgabekammer kann das Verfahren auf vorteilhaft einfache Weise angepasst werden an unterschiedliche Analytkonzentrationen des zu analysierenden Mediums.

Falls das zu analysierende Medium die Analytaufgabekammer nicht kontinuierlich durchströmt, sondern nur zu ausgewählten Zeiten in die Analytaufgabekammer eingeleitet wird, ist es zweckmäßig, nur oder im wesentlichen nur bei Vorliegen des zu analysierenden Mediums in der Analytaufgabekammer frisches Transportmedium in die Analytaufgabekammer einzuleiten.

Die erfindungsgemäße Analysevorrichtung umfasst in weiter bevorzugten Ausführungsformen mehrere Sensoren, wobei die Sensoren einzeln oder in Gruppen in verschiedenen Sensorkammern vorgesehen sein können. Die Sensoren können alle für den gleichen Analyten oder für verschiedene Analyten ausgelegt sein. Die erfindungsgemäße Vorrichtung ermöglicht es daher auf einfache Weise, eine hohe Messgenauigkeit durch Parallelmessungen von Analytkonzentrationen zu erreichen, und außerdem die Konzentrationen mehrerer Analyten gleichzeitig zu bestimmen. Beispielsweise ist es so möglich, gleichzeitig die Glucose- und Laktatkonzentration eines zu analysierenden Mediums zu bestimmen.

In weiter bevorzugten Ausführungsformen enthält die erfindungsgemäße Vorrichtung zwei oder mehr Analytaufgabekammern, optional mit einem oder mehreren Sensoren, wobei die Analytaufgabekammern in Reihe oder gegebenenfalls über einen weiteren Verteiler parallel geschaltet sind. Die verschiedenen Analytaufgabekammern können insbesondere Membranen enthalten, die den Durchtritt unterschiedlicher Analyten aus einem oder verschiedenen zu analysierenden Medien in ein Transportmedium gestatten. Auf diese Weise kann eine besonders gute Abtrennung der zu bestimmenden Analyten erzielt werden.

Wenn die Analytaufgabekammern in Reihe angeordnet sind, wird zum Bestimmen einer Analytkonzentration zunächst Transportmedium aus der ersten Analytaufgabekammer zum Sensor geleitet und dort analysiert. Anschließend wird weiter Transportmedium in Richtung auf den Sensor geleitet, bis das Transportmedium aus der jeweils weiteren Analytaufgabekammer in der Sensorkammer angekommen ist und dort analysiert werden kann.

Bei Verwendung mehrerer parallel geschalteter Analytaufgabekammern wird zunächst eine Analytaufgekammer ausgewählt, zweckmäßigerweise durch einen Verteiler, beispielsweise ein Schiebeventil, aus welcher Analytaufgabekammer Transportmedium in die Sensorkammer übertragen werden soll. Anschließend wird gegebenenfalls analythaltiges Transportmedium aus der Analytaufgabekammer in die Sensorkammer geleitet. Bei Verwendung mehrerer, insbesondere parallel geschalteter Analytaufgabekammern ist es auch möglich, beispielsweise an verschiedenen Stellen eines Bioreaktors eine Analytkonzentration zu bestimmen.

Als Transportmedium wird vorzugsweise ein wässriges Medium verwendet. Das Transportmedium ist zweckmäßigerweise angepasst an den oder die jeweiligen Analyten und den oder die jeweiligen Sensoren. Vorzugsweise enthält das Transportmedium einen pH-Puffer. Vorzugsweise enthält das Transportmedium ferner (a) ein Konservierungsmittel, vorzugsweise Benzoesäure und/oder Propionsäure, (b) ein Tensid, vorzugsweise Triton X und/oder Tween 80, und/oder (c) ein Antikoagulans, vorzugsweise Heparin. Die Verwendung eines heparinhaltigen Transportmediums ist insbesondere bevorzugt für den Fall, dass eine menschliche oder tierische Körperflüssigkeit, insbesondere Blut, analysiert werden soll.

Das zu analysierende Medium ist in bevorzugten Ausführungsformen der Erfindung Blut. Dazu kann einem Patienten oder einem zu behandelnden Tier eine Analytaufgabekammer in Form einer Sonde in ein Blutgefäß eingeführt werden. Die Analytaufgabekammer ist durch eine Zu- und Ableitung mit dem Verteiler und der Sensorkammer einer erfindungsgemäßen Vorrichtung verbunden. Dabei ist die Sensorkammer vorzugsweise dicht an der Analytaufgabekammer angeordnet, wobei es besonders bevorzugt ist, wenn das Volumen der Leitung, durch die Transportmedium zum Bestimmen der Analytkonzentration aus der Analytaufgabekammer in die Sensorkammer strömt, zwischen Analytaufgabekammer und Sensorkammer nicht mehr als das Fünffache des Volumens der Analytaufgabekammer beträgt. Auf diese Weise kann ein rascher Transfer eines analythaltigen, beispielsweise glucosehaltigen, Transportmediums aus der Analytaufgabekammer in die Sensorkammer und eine entsprechend rasche Messung erreicht werden.

Erfindungsgemäß wird auch ein Analyseverfahren gemäß Anspruch 8 bereitgestellt.

Insgesamt bevorzugt ist ein erfindungsgemäßes Analyseverfahren, umfassend das Pumpen eines Transportmediums durch eine Pumpe in eine Pumprichtung, mit den Schritten:
i) Bereitstellen eines zu analysierenden Mediums an der Analytaufgabekammer,
ii) Spülen eines Sensors für die zu analysierende Substanz mit einem Transportmedium in einer ersten Fließrichtung, wobei das Transportmedium nach dem Spülen des Sensors in die Analytaufgabekammer geleitet wird,
iii) Beladen des Transportmediums in der Analytaufgabekammer mit einer Menge des nachzuweisenden Analyten,
iv) ohne Umschalten der Pumprichtung Umkehren der Fließrichtung des Transportmediums und Leiten des ggf. mit Analyten beladenen Transportmedium aus der Analytaufgabekammer zum Sensor,
v) Bestimmen der Analytkonzentration mit dem Sensor in der Sensorkammer,
vi) zum Vorbereiten einer neuen Messung Spülen des Sensors und vorzugsweise auch der Analytaufgabekammer durch Wiederholen von Schritt ii).

Die erfindungsgemäße Analysevorrichtung kann in weiteren bevorzugten Ausführungsformen in einer Bioreaktorsonde angeordnet sein. Dabei kann die Analytaufgabekammer beispielsweise in Form eines membranüberzogenen Kanals wie in den eingangs genannten Patent- und Offenlegungsschriften beschrieben ausgebildet sein. Die Analytaufgabekammer kann jedoch auch in Form eines Shunts oder einer Zu- und Ableitung mit dem Bioreaktor verbunden sein.

Die Analytaufgabekammer ist mit der Sensorkammer über eine Leitung zum Überführen von analytbeladenem Transportmedium zum Bestimmen der Analytkonzentration verbunden, deren Volumen wiederum bevorzugt maximal das Fünffache des Transportmedium-Volumens der Analytaufgabekammer beträgt, um einen raschen Transport des Transportmediums aus der Analytaufgabekammer in die Sensorkammer und entsprechend eine rasche Bestimmung der Analytkonzentration zu ermöglichen.

Ferner ist es bevorzugt, die Analytaufgabekammer und/oder die Sensorkammer durch Temperierungsmittel auf einer vorgewählten Temperatur oder in einem vorgewählten Temperaturbereich zu halten.

Die Erfindung wird nachfolgend anhand der Figuren und eines Ausführungsbeispiels näher beschrieben, ohne dass dadurch der Schutzbereich der Patenansprüche beschränkt werden soll. Es stellen dar:
- Figur 1: ein Fließschema einer erfindungsgemäßen Analysevorrichtung;
- Figur 2: eine schematische Seitenansicht eines kombinierten Verteilers und einer Sensorkammer einer erfindungsgemäßen Vorrichtung;

- Figur 3: eine schematische Draufsicht auf den Verteiler und die Sensorkammer von Figur 2;
- Figur 4: eine schematische Seitenansicht eines Verteilers, einer Sensorkammer und einer Analytaufgabekammer einer erfindungsgemäßen Vorrichtung;
- Figur 5: eine schematische Draufsicht auf den Verteiler, die Analytaufgabekammer und die Sensorkammer der in Figur 4 gezeigte Vorrichtung;
- Figur 6: ein Fließschema der erfindungsgemäßen Analysevorrichtung aus Fig. 4, und
- Figur 7: ein Fließschema einer weiteren erfindungsgemäßen Analysevorrichtung.

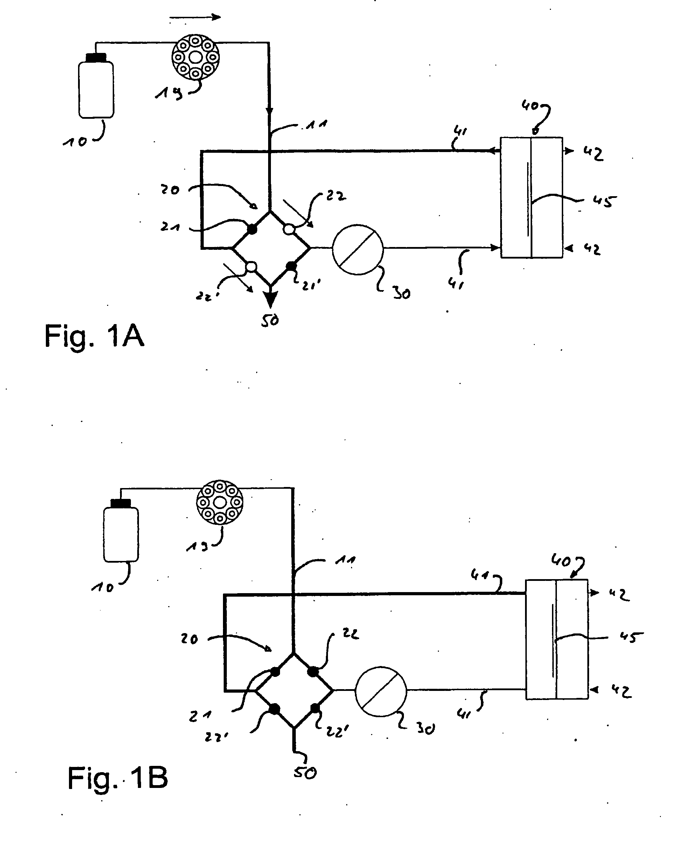

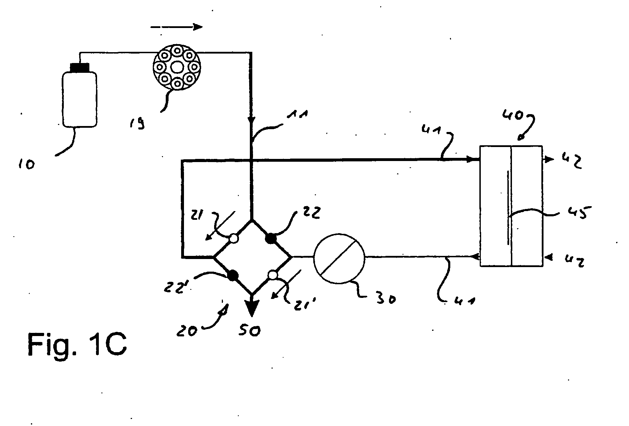

Figur 1 zeigt in drei Teilfiguren A bis C drei Betriebszustände einer erfindungsgemäßen Analysevorrichtung. Die Analysevorrichtung umfasst einen Vorrat 10 einer Transportlösung. Der Vorrat 10 ist über eine Pumpe 19, im dargestellten Ausführungsbeispiel eine Schlauchpumpe, in Fließverbindung verbunden mit einem Verteiler 20. Der Verteiler 20 besitzt einen aus vier Segmenten zusammengesetzten ringförmigen Kanal mit insgesamt vier Schaltpunkten 21, 21', 22, 22'. Die Schaltpunkte 21, 21', 22, 22' sind (hier nicht gezeigt) ausgeführt in Form einer Silikonmembran, die an den jeweiligen Schaltpunkten durch Druckluftbeaufschlagung absperrend in den ringförmigen Kanal hineingepresst werden kann, um ein Durchströmen des jeweiligen Schaltpunktes durch die Transportlösung zu verhindern. An den Stellen, an denen jeweils zwei der vier Segmente des ringförmigen Kanals aneinander stoßen, sind Zu- bzw. Ablaufkanäle vorgesehen, und zwar (im Uhrzeigersinn) eine Zuleitung 11 von Pumpe 19, zu einer Sensorkammer 30, zu einem Auslass 50 und zu einer Analytaufgabekammer 40. Die Sensorkammer 30 ist zum Einen verbunden mit dem Verteiler 20 und zum Anderen fließfähig verbunden mit einer Zuleitung 41 der Analytaufgabekammer 40, deren Ableitung 41 für Transportlösung wie gesagt verbunden ist mit dem Verteiler 20. Die Analytaufgabekammer 40 umfasst neben der Zu- und Ableitung 41 für Transportlösung und eine hiervon durch eine Membran 45 abgetrennte Zu- und Ableitung 42 für ein zu analysierendes Medium. Die Membran 45 ist in der Lage, den Analyten von dem zu analysierenden Medium abzutrennen. In bevorzugten Ausführungsformen wird eine Membran 45 verwendet, die Moleküle mit einer Größe von über 12000 Da auf der Seite des zu analysierenden Mediums zurückhält und lediglich den Durchtritt für Analyten einer Größe von bis zu 12000 Da in das Transportmedium erlaubt.

Die Sensorkammer 30 enthält einen Sensor zum Nachweisen des Analyten, vorzugsweise einen Biosensor, beispielsweise eine immobilisierte Glucoseoxidase auf einer amperometrischen Elektrode.

Zum Spülen der erfindungsgemäßen Vorrichtung (vgl. Fig. 1A) werden die Schaltpunkte 21 und 21' des Verteilers 20 durch Druckluftbeaufschlagung geschlossen. Transportmedium wird über die Pumpe 19 aus dem Vorrat 10 durch den offenen Schaltpunkt 22 in die Sensorkammer 30, durch die Analytaufgabekammer 40 und von der Analytaufgabekammer 40 zurück in den Verteiler 20 geleitet. Von dort gelangt es durch den geöffneten Schaltpunkt 22' aus dem Verteiler heraus in den Auslass 50 und wird verworfen. Auf diese Weise wird die Sensorkammer 30 und die Analytaufgabekammer 40 auf Seiten des Transportmediums gespült und der Sensor (nicht dargestellt) der Sensorkammer 30 von eventuell bisher im Transportmedium enthaltenen Analyten gereinigt. Auf diese Weise kann ein Nullabgleich des Sensors bewirkt werden. Während des Spülens der Sensorkammer 30 und der Analytaufgabekammer 40 kann die Analytaufgabekammer auf Seiten des zu untersuchenden Mediums weiter von einem zu untersuchenden Medium durchströmt werden, eine solche Durchströmung kann aber auch unterbunden werden. Da während des Spülens kein Transportmedium aus der Analytaufgabekammer 40 in die Sensorkammer 30 gelangen kann, ist ein Unterbrechen der Zugabe des zu analysierenden Mediums jedoch nicht erforderlich.

In Figur 1B ist eine Analytsammelphase der erfindungsgemäßen Vorrichtung gezeigt. Die Pumpe 19 ist abgeschaltet, sodass kein Transportmedium durch die Analytaufgabekammer 40 fließt. Diese Analytaufgabekammer 40 wird auf der Seite des zu untersuchenden Mediums nunmehr mit zu untersuchendem Medium beaufschlagt, beispielsweise indem die Analytaufgabekammer 40 auf dieser Seite von dem zu untersuchenden Medium durchströmt wird. Durch die Membran der Analytaufgabekammer 40 tritt der Analyt aus dem zu untersuchenden Medium in das Transportmedium über.

Figur 1C zeigt eine Messung mit einer erfindungsgemäßen Vorrichtung. Dazu werden die Schaltpunkte 21, 21' des Verteilers 20, geöffnet und die Schaltpunkte 22, 22' des Verteilers 20 geschlossen, vorzugsweise wie oben beschrieben durch Beaufschlagung einer Silikonmembran mit Druckluft. Aus dem Vorrat 10 pumpt die Pumpe 19 Transportmedium durch die Zuleitung 11 in den Verteiler 20, von wo aus das Transportmedium durch den geöffneten Schaltpunkt 21 durch die Zuleitung 41 in die Analytaufgabekammer 40 strömt. Das in der Analytaufgabekammer 40 enthaltene, analytenbeladene Transportmedium wird aus der Analytaufgabekammer 40 heraus durch die Ableitung 41 in die Sensorkammer 30 geleitet. Das bisher in der Sensorkammer 30 vorliegende Transportmedium wird aus der Sensorkammer 30 in den Verteiler 20 und von dort aus durch den geöffneten Schaltpunkt 21' in den Auslass 50 befördert. In der Sensorkammer 30 wird dann das Vorliegen des Analyten und vorzugsweise seine Konzentration durch den Sensor bestimmt. Der Sensor gibt dann ein der bestimmten Größe entsprechendes Signal an eine Ausgabe- oder Verarbeitungsvorrichtung (nicht dargestellt) ab, vorzugsweise an eine Anzeige zum Anzeigen der Analytkonzentration.

Wenn das Vorliegen des Analyten oder seine Konzentration durch den Sensor der Sensorkammer 30 bestimmt wurde, kann die Sensorkammer 30 erneut wie zuvor mit Bezug auf Figur 1A beschrieben mit Transportmedium durchspült werden. Falls ein solches Durchspülen nicht gewünscht ist, kann auch in dem in Figur 1C dargestellten Schaltzustand weiter Transportmedium über die Pumpe 19 und dem Verteiler 20 durch die Probenaufgabekammer 40 in die Messzelle 30 verbracht werden. Auf diese Weise ist ein gleichmäßiges Durchströmen der Sensorkammer 30 mit analytbeladenem Transportmedium und eine fortgesetzte Überwachung der Anwesenheit und der Konzentration des Analyten möglich. Während des gesamten Betriebs der Vorrichtung bleibt das zu untersuchende Medium vollständig getrennt von dem Transportmedium, und ebenfalls bleibt das Transportmedium bis auf die Substanzen, die die Membranen der Analytaufgabekammer passieren können, von dem zu analysierenden Medium getrennt. Die erfindungsgemäße Analysevorrichtung ist dementsprechend besonders geeignet zum Analysieren von Medien, die steril bleiben müssen, oder von denen außer der zu analysierenden Substanz und kleineren Substanzen keine anderen Substanzen nach Außen gelangen sollen.

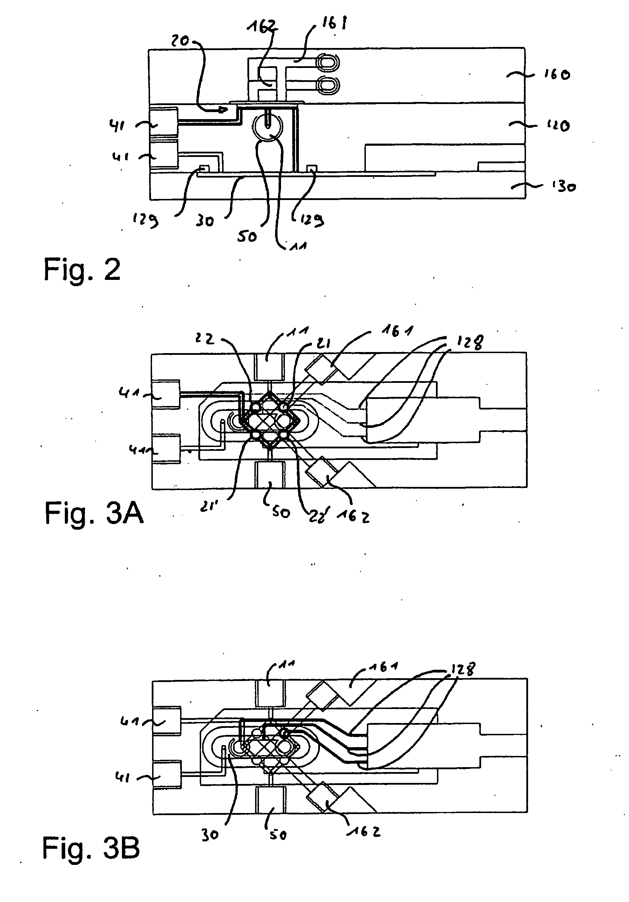

Figur 2 zeigt nun eine schematische Seitenansicht einer Kombination eines Verteilers und einer Sensorkammer einer erfindungsgemäßen Vorrichtung, während Figur 3 in zwei Teilfiguren 3A und 3B eine schematische Draufsicht auf die in Figur 2 gezeigte Baugruppe zeigt. In den Figuren 2 und 3A ist der Verteiler 20 mit den Schaltpunkten 21, 21', 22, 22' entsprechend dem Fließschema der Figur 1A durch dicke Strichstärken hervorgehoben. In Figur 3B sind elektrische Verbindungen zur Sensorkammer durch dicke Strichstärken hervorgehoben. Die in den Figuren 2 und 3 dargestellte Baugruppe umfasst keine Analytaufgabekammer 40, sondern Anschlüsse der Zu- und Ableitungen 41 zum Anschließen einer (externen) Analytaufgabekammer 40 Die Baugruppe umfasst einen Sensorkammer-Grundkörper 130. Der Sensorkammer-Grundkörper 130 steht flüssigkeitsdicht in Kontakt mit einem Kanal-Grundkörper 120, der insbesondere die Kanäle des Verteilers 20 (vgl. Fig. 1) und die zugehörigen Zu- und Ableitungen zur Sensorkammer 30 und zur Analytaufgabekammer 40 enthält. Der ringförmige Kanal des Verteilers 20 ist an den Schaltpunkten 21, 21', 22, 22' über eine Membran druckschlüssig verbunden mit zwei Druckkanälen 161, 162 in einem Schalt-Grundkörper 160.

Die Kanäle des Kanal-Grundkörpers 120 sind mit entsprechenden Kanälen und Bohrungen des Schalt-Grundkörpers 160 und des Sensorkammer-Grundkörpers 130 wirksam verbunden. Im Betrieb der in den Figuren 2 und 3 dargestellten Baugruppe wird ein Transportmedium, beispielsweise über eine Pumpe 19 (dargestellt in Fig. 1), durch eine Zuleitung 11 in den ringförmigen Kanal des Verteilers 20 des Kanal-Grundkörpers 120 eingeleitet. Durch Beaufschlagen des Druckkanals 161 mit Druckluft wird an den Schaltpunkten 21, 21' der ringförmige Kanal flüssigkeitsdicht geschlossen. Das Transportmedium strömt dann durch den ringförmigen Kanal des Verteilers 20 und eine Zuleitung 41 bis zu einem Auslass in Richtung auf die Analytaufgabekammer 40. Von der Analytaufgabekammer 40 rückfließendes Transportmedium gelangt durch einen weiteren Kanal 41 des Kanal-Grundkörpers 120 in die Sensorkammer des Sensorkammer-Grundkörpers 130. Die Sensorkammer des Sensorkammer-Grundkörpers 130 ist mit dem Schalt-Grundkörper 120 über eine Dichtung 129 abdichtend verbunden, so dass ein Austreten von Transportmedium in einen eventuell bestehenden Spalt zwischen dem Schalt -Grundkörper 120 und dem Sensorkammer-Grundkörper 130 vermieden wird.

In der Sensorkammer ist ein Biosensor angeordnet, beispielsweise eine amperometrische Elektrode in Wirkverbindung mit immobilisierter Glucoseoxidase zum Bestimmen einer Glucosekonzentration in dem Transportmedium. Der Sensor der Sensorkammer 30 erzeugt ein elektrisches Signal, das über elektrische Verbindungen 128 abgeführt wird. Die elektrischen Verbindungen können im Schalt-Grundkörper und/oder im Sensorkammer-Grundkörper 130 vorgesehen sein. Aus der Sensorkammer 30 gelangt das Transportmedium durch einen Kanal des Kanal-Grundkörpers 120 in den ringförmigen Kanal des Verteilers 20 und von dort durch den geöffneten Schaltpunkt 22' und einen zugehörigen Auslass 50 aus der Baugruppe hinaus, beispielsweise in einen Abfallsammelbehälter.

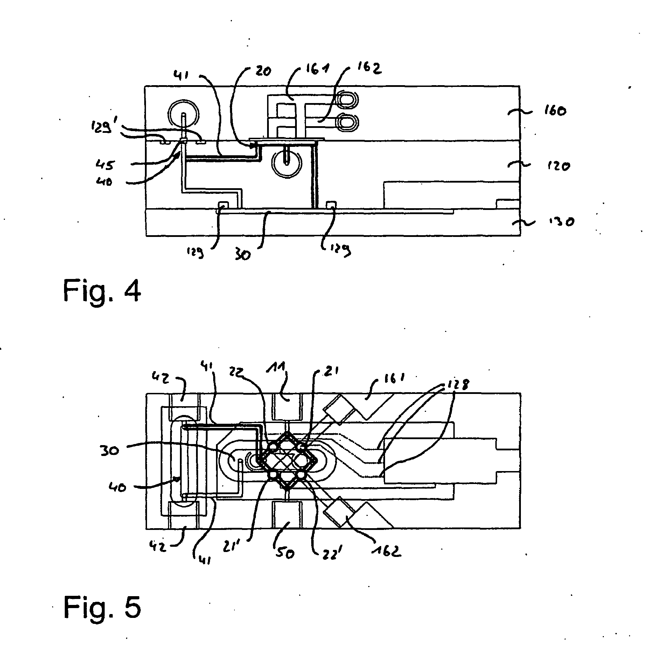

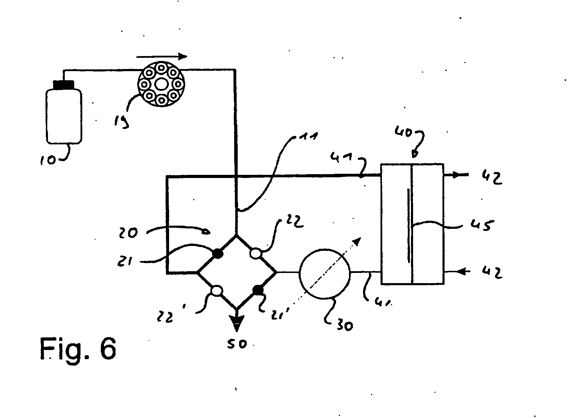

Die Baugruppe gemäß der Figuren 4 und 5 unterscheidet sich von der der Figuren 2 und 3 lediglich darin, dass die Analytaufgabekammer 40 Teil der Baugruppe ist. Figur 6 zeigt einen der Figur 1A entsprechenden Schaltzustand der Baugruppe gemäß Figuren 4 und 5, wobei wiederum hier und in den Figuren 4 und 5 der Verteiler 20 und die Verbindung der Analytaufgabekammer 40 zum Verteiler 20 durch dicke Strichstärke hervorgehoben sind. Zum Bilden der Analytaufgabekammer 40 ist im Kanal-Grundkörper 120 ein Transportmedium-Kanal und auf der gegenüberliegenden Seite im Schalt-Grundkörper 160 ein Kanal für zu analysierendes Medium vorgesehen. Beide Kanäle sind durch eine Membran 45 voneinander getrennt, die den Durchtritt einer zu analysierenden Substanz aus dem zu analysierenden Medium in das Transportmedium gestattet. Vorzugsweise wird eine Membran verwendet, die Moleküle mit einer Größe von über 12000 Da auf der Seite des zu analysierenden Mediums zurückhält und lediglich Molekülen von 12000 Da und weniger den Durchtritt in das Transportmedium ermöglicht. Die Analytaufgabekammer 40 ist gegen das Austreten von zu analysierendem Medium und von Transportmedium in einen eventuell vorhandenen Spalt zwischen dem Schalt-Grundkörper 160 und dem Kanal-Grundkörper 120 durch eine Dichtung 129' geschützt. Der Schalt-Grundkörper enthält, unverbunden mit den Druckkanälen 161, 162, Anschlüsse zum Aufgeben eines zu analysierenden Mediums auf die Analytaufgabekammer 40. Die Baugruppe der Figuren 4 und 5 wird entsprechend betrieben wie die Baugruppe der Figuren 2 und 3.

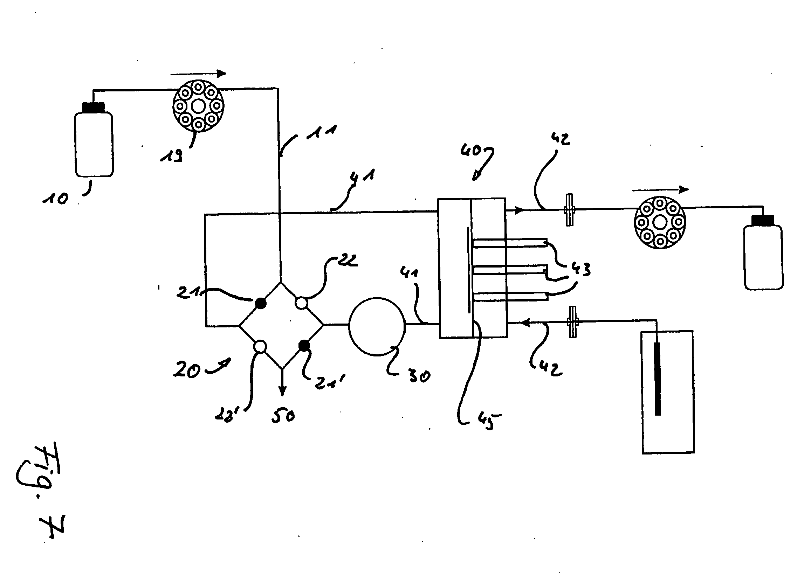

Figur 7 zeigt eine weitere Analysevorrichtung der in Figur 1 dargestellten Art. Die Analysevorrichtung besitzt zusätzlich drei Sensoren 43. Die Sensoren 43 sind in der Analytaufgabekammer 40 angeordnet, im dargestellten Fall auf der Seite des zu analysierenden Mediums. Das zu analysierende Medium wird aus einer Quelle entnommen, beispielsweise einem Bioreaktor oder einem Blutgefäß. Es wird über einen Sterilfilter zur Analytaufgabekammer 40 und den dort angeordneten Sensoren 43 geleitet. Aus der Analytaufgabekammer 40 wird es über einen weiteren Sterilfilter und eine Pumpe in eine Senke, beispielsweise ein Abfallauffanggefäß kontinuierlich oder zu ausgewählten Zeitpunkten gepumpt. Ansonsten wird die Analysevorrichtung wie oben in bezug auf Figur 1 beschrieben betrieben. Insbesondere besitzt die Analysevorrichtung einen Verteiler 20 und eine Sensorkammer 30, die wie oben beschrieben mit Transportmedium beaufschlagt werden können.

## Patentansprüche

1. Analysevorrichtung, umfassend
a) eine Sensorkammer 30 mit einem Sensor zum Detektieren eines Analyten in einem Transportmedium.
b) eine Analytaufgabekammer 40 zum Aufnehmen des Analyten in dem Transportmedium,
c) einen Verteller 20 in Fließverbindung mit der Sensorkammer 30 und der Analytaufgabekammer 40, und
d) eine Steuerung zum Steuern eines Transportmedium-Stromes,
wobei der Verteil 20 umfasst;
(c1) einen ringförmien Kanal mit vier beabstandet voneinander angeordneten Anschlüssen,
wobei
(c₂) der Verteiler 20 jeweils eine schaltbare Absperrung 21,21',22,22' zwischen zwei benachbarten Anschlüssen umfasst,
und dass die Steuerung eingerichtst ist zum Steuern des Vertellers 20 dergestalt,
dass
- zum Spülen das Transportmedium vom Verteller 20 ohne Passieren der Analytaugabekammer 40 in die Sensorkammer 30 geführt wird, und
- zum Messen das Transportmedium vom Verteller 20 unter Passieren der Analytaufgabekammer 40 in die Sensorkammer 30 geführt wird, **dadurch gekennzeichnet, dass** die Sensorkammer (30) fließfähig verbunden ist mit einer Zuleitung 41 der Analytaufgabekammer 40.

2. Analysevorrichtung nach Anspruch 1, wobei jeds der schaltbaren Absperrungen 21,21',22,22' zwischen zwei benachbarten Anschlüssen ein durch Druck eines Steuerfluids oder Magnetismus schaltbares Ventil ist.

3. Analysevorrichtung nach einem der vorherigen Ansprüche, wobei der Sensor ausgewählt ist aus der Gruppe bestehend aus einem elektrochemischen oder optischen Sensor.

4. Analysevorrichtung nach einem der vorherigen Ansprüche, **dadurch gekennzeichnet, dass** die Analytaufgabekammer 40 mit einem zu analysierenden Medium verbunden ist über eine Membran 45, die den Durchtritt des Analyten aus dem zu analysierenden Medium in das Transportmedium ermöglicht.

5. Analysevorrichtung nach einem der vorherigen Ansprüche, **dadurch gekennzeichnet, dass** die Sensorkammer 30 und der Verteiler 20 in einem einheitlichen Baukörper enthalten sind.

6. Analysevorrichtung nach einem der vorherigen Ansprüche, **dadurch gekennzeichnet, dass** die Sensorkammer 30 zwei, drei oder mehr Sensoren enthält.

7. Analysevorrichtung nach einem der vorherigen Ansprüche, **dadurch gekennzeichnet, dass** die Analytaufgabekammer 40 einen, zwei, drei oder mehr Sensoren enthält.

8. Analyseverfahren für eine Analysevorrichtung nach einem der Ansprüche 1 bis 7, umfassend das Pumpen eines Transportmediums durch eine Pumpe 19 in eine Pumprichtung, mit den Schritten:
- Spülen der Sensors kammer 20 für die zu analysierende Substanz mit dem Transportmedium in einer ersten Fließhchtung, wobei das Transportmedium nach dem Spülen der Sensor kammer 40 in eine Analytaufgabekammer 40 geleitet wird,
- Beladen des Transportmediums in der Analytaufgabekammer 40 mit einer Menge des nachzuweisenden Analyten, und
- ohne Umschalten der Pumprichtung Umkehren der Fließrichtung des Transportmediums, um das ggf. mit Analyten beladene Transportmedium aus der Analytaufgabekammer 40 zur Sensor krammer 20 zu leiten.

9. Verwendung einer Analysevorrichtung nach einem der Ansprüche 1 bis 7 oder eines Analyseverfahren nach Anspruch 8 zum Bestimmen einer Analytkonzentration, insbesondere der Konzentration von Glucose, Saccharose. Glycerin, Methanol, Glutamin, Glutamat, Phosphat, Lactat, Lactose, Ammonium, Ethanol, eines Antikörpers, Pestizids, Antibiotikums, Proteingehalts, Fettgehalts, Wassergehalts, pO₂, pCO₂ und/oder pH.

## Claims

1. An analytical device, comprising
a. a sensor chamber (30) with a sensor for detecting an analyte in a transport medium,
b. an analyte feed chamber (40) for receiving the analyte in the transport medium,
c. a distributor (20) in flow connection with the sensor chamber (30) and with the analyte feed chamber (40), and
d. a control means to control a transport-medium flow,
wherein the distributor (20) comprises:
c1) an annular channel with four connections arranged spaced apart from one another,
wherein
c2) the distributor (20) in each case comprises an actuable shut-off device (21, 21', 22, 22') between two adjacent connections,
and in that the control means is configured to control the distributor (20) in such a manner that
- for rinsing, the transport medium is guided into the sensor chamber (30) from the distributor (20) without passing through the analyte feed chamber (40), and
- for measuring, the transport medium is guided into the sensor chamber (30) from the distributor (20) while passing through the analyte feed chamber (40),
**characterised in that** the sensor chamber (30) is connected in a free-flowing manner to a feed line (41) of the analyte feed chamber (40).

2. An analytical device according to claim 1, wherein each of the actuable shut-off devices (21, 21', 22, 22') between two adjacent connections is a valve actuable by pressure of a control fluid or by magnetism.

3. An analytical device according to any one of the preceding claims, wherein the sensor is selected from the group consisting of an electrochemical or optical sensor.

4. An analytical device according to any one of the preceding claims, **characterised in that** the analyte feed chamber (40) is connected via a membrane (45) to a medium to be analysed, which membrane enables the passage of the analyte from the medium to be analysed into the transport medium.

5. An analytical device according to any one of the preceding claims, **characterised in that** the sensor chamber (30) and the distributor (20) are contained in a unitary structure.

6. An analytical device according to any one of the preceding claims, **characterised in that** the sensor chamber (30) contains two, three or more sensors.

7. An analytical device according to any one of the preceding claims, **characterised in that** the analyte feed chamber (40) contains one, two, three or more sensors.

8. An analytical method for an analytical device according to any one of claims 1 to 7, comprising the pumping of a transport medium through a pump (19) in a pumping direction, having the steps:
- rinsing of the sensor chamber (20) for the substance to be analysed with the transport medium in a first flow direction, wherein the transport medium is led into an analyte feed chamber (40) after rinsing of the sensor chamber (20),
- charging of the transport medium in the analyte feed chamber (40) with a quantity of the analyte to be traced, and
- reversal of the flow direction of the transport medium without changing over of the pumping direction, in order to lead the transport medium, optionally charged with analyte, from the analyte feed chamber (40) to the sensor chamber (20).

9. Use of an analytical device according to any one of claims 1 to 7 or of an analytical method according to claim 8 for determining an analyte concentration, in particular the concentration of glucose, saccharose, glycerol, methanol, glutamine, glutamate, phosphate, lactate, lactose, ammonium, ethanol, an antibody, pesticide, antibiotic, protein content, fat content, water content, p0_{2.} pC0₂, and/or pH.

## Revendications

1. Dispositif d'analyse, comprenant
a) une chambre de capteur 30 avec un capteur pour la détection d'un analyte dans un milieu de transport,
b) une chambre d'alimentation en analyte 40 pour le logement de l'analyte dans le milieu de transport,
c) un répartiteur 20 en liaison fluidique avec la chambre de capteur 30 et la chambre d'alimentation en analyte 40, et
d) une commande pour la commande d'un flux de milieu de transport, le répartiteur 20 comprenant :
(c1) un canal de forme annulaire avec quatre branchements disposés à distance les uns des autres,
(c2) le répartiteur 20 comprenant à chaque fois un dispositif de blocage (21, 21', 22, 22') commutable entre deux branchements voisins,
et en ce que la commande est aménagée pour la commande du répartiteur 20, de telle sorte que
- pour le lavage, le milieu de transport est guidé depuis le répartiteur 20, sans passer par la chambre d'alimentation en analyte 40, dans la chambre de capteur 30, et
- pour la mesure, le milieu de transport est guidé depuis le répartiteur 20, en passant par la chambre d'alimentation en analyte 40, dans la chambre de capteur 30, **caractérisé en ce que** la chambre de capteur (30) est reliée de façon fluidique à une arrivée 41 de la chambre d'alimentation en analyte 40.

2. Dispositif d'analyse selon la revendication 1, chacun des dispositifs de blocage (21, 21', 22, 22') entre deux branchements voisins étant une vanne commutable par pression d'un fluide de commande ou magnétisme.

3. Dispositif d'analyse selon l'une quelconque des revendications précédentes, le capteur étant choisi dans le groupe comprenant un capteur électrochimique et un capteur optique.

4. Dispositif d'analyse selon l'une quelconque des revendications précédentes, **caractérisé en ce que** la chambre d'alimentation en analyte 40 est reliée à un milieu à analyser par une membrane 45, qui permet le passage de l'analyte du milieu à analyser dans le milieu de transport.

5. Dispositif d'analyse selon l'une quelconque des revendications précédentes, **caractérisé en ce que** la chambre de capteur 30 et le répartiteur 20 sont intégrés dans un corps homogène.

6. Dispositif d'analyse selon l'une quelconque des revendications précédentes, **caractérisé en ce que** la chambre de capteur 30 contient deux, trois ou plus de trois capteurs.

7. Dispositif d'analyse selon l'une quelconque des revendications précédentes, **caractérisé en ce que** la chambre d'alimentation en analyte 40 contient un, deux, trois ou plus de trois capteurs.

8. Procédé d'analyse pour un dispositif d'analyse selon l'une quelconque des revendications 1 à 7, comprenant le pompage d'un milieu de transport par une pompe 19 dans un sens de pompage, comprenant les étapes suivantes :
- lavage de la chambre de capteur 20 pour la substance à analyser avec le milieu de transport dans un premier sens d'écoulement, le milieu de transport étant acheminé après le lavage de la chambre de capteur 20 dans une chambre d'alimentation en analyte 40,
- chargement du milieu de transport dans la chambre d'alimentation en analyte 40 avec une quantité de l'analyte à mettre en évidence, et
- sans commutation du sens de pompage, inversion du sens d'écoulement du milieu de transport, afin d'acheminer le milieu de transport chargé éventuellement avec des analytes de la chambre d'alimentation en analyte 40 à la chambre de capteur 20.

9. Utilisation d'un dispositif d'analyse selon l'une quelconque des revendications 1 à 7 ou d'un procédé d'analyse selon la revendication 8 pour déterminer une concentration d'analyte, en particulier la concentration de glucose, saccharose, glycérine, méthanol, glutamine, glutamate, phosphate, lactate, lactose, ammonium, éthanol, d'un anticorps, pesticide, antibiotique, d'une teneur en protéine, d'une teneur en graisse, d'une teneur en eau, du pO₂, du pCO₂ et/ou du pH.
